# EUROPÄISCHE PATENTSCHRIFT

(11) **EP 0 912 470 B1**
(45) Veröffentlichungstag und Bekanntmachung des Hinweises auf die Patenterteilung: **13.02.2002**
(21) Anmeldenummer: 97931688.2
(22) Anmeldetag: 28.06.1997
(51) Int. Cl.: C07B 63/00, C07H 1/06, C07H 19/20, C07H 21/00, C07K 1/14, C25B 3/00

(54) **VERFAHREN UND VORRICHTUNG ZUR AUFREINIGUNG UND ANREICHERUNG VON MOLEKÜLEN**
METHOD AND DEVICE FOR PURIFYING AND ENRICHING MOLECULES
PROCEDE ET DISPOSITIF DE PURIFICATION ET D'ENRICHISSEMENT DE MOLECULES

(30) Priorität: 12.07.1996 DE 19628171; 10.02.1997 DE 29702254 U
(43) Veröffentlichungstag der Anmeldung: 06.05.1999
(73) Patentinhaber: november Aktiengesellschaft Gesellschaft für Molekulare Medizin, 91056 Erlangen (DE)
(72) Erfinder: Bertling, Wolf M., Prof. Dr., 91056 Erlangen (DE)
(74) Vertreter: Gassner, Wolfgang, Dr.
(86) Internationale Anmeldenummer: DE9701368
(87) Internationale Veröffentlichungsnummer: WO9802399

(56) Entgegenhaltungen:
- EP-A- 0 031 565
- WO-A-89/05454
- WO-A-94/25144
- FR-A- 2 687 931
- US-A- 4 545 888
- US-A- 4 715 942
- US-A- 4 915 811
- US-A- 5 151 165
- US-A- 5 196 099
- US-A- 5 217 593

## Beschreibung

Die Erfindung betrifft ein Verfahren und eine Vorrichtung zur Aufreinigung und Anreicherung von ladungstragenden ersten Molekülen wie Proteinen, Nukleinsäuren und dgl..

Die Anreicherung von biologisch relevanten Molekülen, wie Nukleinsäuren zur Durchführung analytischer Tests, bsp. der "polymerase chain reaction" (PCR), kann durch Fällen der Nukleinsäuren, durch Binden der Nukleinsäuren an eine geeignete Matrix, bsp. unter Verwendung einer Ionenaustauscher-Säule, sowie durch verschiedene Zentrifugationsverfahren erfolgen. Die Selektion spezifischer Nukleinsäuren aus einem Nukleinsäuregemisch kann durch Anreicherung und Abtrennung in einem Gel, durch Hybridisation an Membranen oder durch das Komplexieren mit spezifischen Proteinen erfolgen. - Zur Aufreinigung von Proteinen werden ähnliche Verfahren herangezogen; dabei kommen auch Verfahren wie die Hochdruckflüssigkeitschromatographie (HPLC) sowie antikörperabhängige Aufreinigungsverfahren zur Anwendung. Antikörperabhängige Verfahren verwenden oberflächenfixierte Moleküle, wie z.B. Latex-beads. - Die bekannten Verfahren haben den Nachteil mangelnder Sensitivität und Geschwindigkeit. Außerdem ist deren Durchfuhrung kostenaufwendig.

Der vorliegenden Erfindung liegt die Aufgabe zugrunde, ein Aufreinigungs- und Anreicherungsverfahren sowie eine entsprechende Vorrichtung bereitzustellen, mit denen die Nachteile nach dem Stand der Technik beseitigt werden. Außerdem sollen insbesondere Nukleinsäuren, Proteine und andere ladungstragende Moleküle von vorgegebenem Homologiegrad bzw. vorgegebener Bindungsaffinität aus einem großen Volumen anreicherbar sein.

Diese Aufgabe wird durch die Merkmale der Patentansprüche 1 und 13 gelöst. Zweckmäßige Weiterbildungen ergeben sich aus den Merkmalen der Patentansprüche 2 - 12 sowie 14 - 35.

Nach der verfahrensseitigen Maßgabe der Erfindung wird die Aufgabe durch die folgenden Schritte gelöst:
a) Bereitstellen einer die ersten Moleküle enthaltenden Lösung,
b) Inkontaktbringen der Lösung mit mindestens einer Elektrode, auf der unmittelbar eine Beschichtung mit eine Affinität zu den ersten Molekülen aufweisenden zweiten Molekülen vorgesehen ist und
c) Verbinden der Elektrode mit einem Mittel zur Erzeugung eines elektrischen Felds, so daß in der Lösung eine in bezug zur Beschichtung gerichtete Bewegung der ersten Moleküle bewirkt wird.

Die Elektrode ist zweckmäßigerweise aus einem elektrisch leitfähigen Kuststoff hergestellt. Sie kann eine Schicht auf einem elektrisch nichtleitenden Kunststoffträgerstab oder ein, vorzugsweise endständiger, Abschnitt eines solchen Kunststoffträgerstabs sein. Es ist auch möglich, den Kunstoffträgerstab vollständig aus elektrisch leitfähigem Kunststoff herzustellen und mit einem aus elektrisch nichtleitenden Kunststoff hergestellten Griffelement zu versehen, das bspw. aufgesteckt sein kann.

Unter Einwirkung des elektrischen Felds wird der Effekt ausgenutzt, daß die in Losung befindlichen ersten Moleküle, z.B. Nukleinsaure-Moleküle, eine Ladung tragen und somit im elektrischen Feld bewegbar sind. Die durch die Einwirkung des elektrischen Felds mit der Beschichtung in Kontakt getretenen oder in unmittelbare Nähe gelangten zweiten Moleküle können dort an die ersten Moleküle gebunden werden. Dabei kann als Bindung insbesondere eine ionische, kovalente, Wasserstoffbrücken- oder durch sterische Einflüsse hervorgerufene Bindung in Frage kommen. Während der Ausbildung dieser Bindung muß kein elektrisches Feld anliegen.

Das erfindungsgemäße Verfahren ist nicht nur dann anwendbar, wenn die ersten Moleküle sich in einer Lösung befinden. Es genügt, wenn sich die ersten Moleküle in einer Matrix, bspw. Gel, Fleisch oder dgl., sich befinden, die im elektrischen Feld eine Wanderung derselben ermöglicht.

Nach einer Ausgestaltung der Erfindung wird zur Erzeugung des elektrischen Felds eine erste Spannung an die Elektrode angelegt, so daß sie als Anode wirkt, an die eine negative Ladung tragende erste Moleküle, z.B. Nukleinsäure-Moleküle, so angezogen werden, daß eine Bindung an die zweiten Moleküle erreicht wird. Nach einer alternativen Ausgestaltung wird zur Erzeugung des elektrischen Felds eine erste Spannung an die Elektrode angelegt, so daß sie als Kathode wirkt, an die eine positive Ladung tragende erste Moleküle, z.B. Proteine, so angezogen werden, daß eine Bindung an die zweiten Moleküle erreicht wird.

Als besonders vorteilhaft wird angesehen, wenn insbesondere nach dem Binden der ersten an die zweiten Moleküle der folgende Schritt durchgeführt wird:
d₁) Umpolen und Anlegen einer zweiten Spannung, so daß die Elektrode als Kathode wirkt, von der eine negative Ladung tragende erste Moleküle, z.B. Nukleinsäuren, abgestoßen werden. Alternativ dazu kann insbesondere nach dem Binden der ersten an die zweiten Moleküle der folgende Schritt durchgeführt werden:
d₂) Umpolen und Anlegen einer zweiten Spannung, so daß die Elektrode als Anode wirkt, von der eine positive Ladung tragende erste Moleküle, z.B. Proteine, abgestoßen werden.

Durch die Schritte lit. d₁) und d₂) kann ein Zusetzen der Beschichtung verhindert werden. Durch eine geeignet gewählte zweite Spannung ist es nämlich möglich, nicht an die Beschichtung gebundene Ladungsträger von der Beschichtung abzustoßen und so den Zugang für weitere erste Moleküle zu verbessern. Durch eine geeignete Erhöhung der zweiten Spannung können außerdem gezielt bestimmte ladungstragende erste Moleküle von der Beschichtung entfernt werden. Auf diese Weise kann auch eine Selektion bestimmter erster Moleküle erreicht werden. Dieser Vorgang ist als Stringenz einer Hybridisationsreaktion für Nukleinsäuren bekannt.

Bei der Umpolung ist zu beachten, daß die Wanderung derjenigen ersten Moleküle, die eine Wechselwirkung mit den zweiten Molekülen eingegangen sind bzw. mit diesen hybridisieren, eingeschränkt ist. Der Grad dieser Behinderung ist von der Art und Anzahl der Wechselwirkungen, d.h. in erster Linie vom Grad der Homologie, der miteinander wechselwirkenden ersten und zweiten Moleküle bzw. deren Affinität abhängig. Erste Moleküle, die eine hohe Affinität bzw. Komplementarität zu den zweiten Molekülen aufweisen, werden dabei am stärksten zurückgehalten.

Zweckmäßigerweise wird während und/oder nach dem Schritt lit. c der folgende Schritt durchgefuhrt:
e) Aufheizen der Elektrode oder der Lösung, so daß die ersten Moleküle thermisch in ihre Komponenten oder Untereinheiten, z.B. einzelsträngige Nukleinsäuren, dissoziiert oder denaturiert werden.

Diese Ausgestaltung ermöglicht bsp. das Aufschmelzen von an die Beschichtung angezogenen doppelsträngigen Nukleinsäuren und erleichtert somit die Bindung der Einzelstränge an bspw. in der Beschichtung vorgesehene komplementäre Oligonukleotide. Auch das Halten der Oberfläche bei einer bestimmten Temperatur leistet somit einen Beitrag zur Stringenz der Selektion bestimmter bindender erster Moleküle.

Zweckmäßigerweise wird insbesondere nach dem Schritt lit. d₁ bzw. d₂ der folgende Schritt durchgeführt:
f) Abkühlen der Elektrode oder der Lösung, so daß eine Bindung der ersten Moleküle, Komponenten und/oder Untereinheiten derselben an die zweiten Moleküle bewirkt wird.

Durch den Schritt lit. f wird die Bindung der ersten Moleküle, deren Komponenten oder Untereinheiten an die Beschichtung zusätzlich unterstützt.

Je nachdem welcher Art die ersten Moleküle sind, kann/können einer oder mehrere der Schritte lit. c - lit. e wiederholt werden.

Insbesondere während und/oder nach dem Schritt lit. d₁ bzw. d₂ ist es zweckmäßig, die Lösung vorzugsweise mechanisch zu durchmischen. Auf diese Weise wird die Entfernung von durch die Schritte lit. d₁ bzw. d₂ von der Elektrode abgestoßenen ersten Molekülen, Komponenten und/oder Untereinheiten unterstützt.

Vorteilhafterweise werden die Maximalwerte der ersten und zweiten Spannung so gewählt, daß eine Degradierung durch Elektrolyse der ersten und zweiten Moleküle sowie der Komponenten und/oder Untereinheiten vermieden wird. Daneben hat es sich als günstig erwiesen, den Maximalwert der zweiten Spannung so zu wählen, daß eine Trennung von zwischen den ersten Molekülen bzw. deren Komponenten oder Untereinheiten und den zweiten Molekülen gebildeten Bindungen vermieden wird.

In Anpassung an die Art des anzureichernden bzw, aufzureinigenden ersten Moleküls können die Maximalwerte der ersten und/oder zweiten Spannung/en, die Dauer der Polung bzw. Umpolung und/oder die Temperatur der Elektrode in Abhängigkeit von Parametern der Lösung, wie deren ph-Wert, Ionenleitfähigkeit, Konzentration an ersten Molekülen, Temperatur und dgl. vorzugsweise automatisch gesteuert werden.

Nach der vorrichtungsseitigen Lösung ist eine Vorrichtung zur Anreicherung von ladungstragenden in einer Lösung befindlichen ersten Molekülen, wie Nukleinsäuren, Proteinen und dgl., mit einer Elektrode vorgesehen, auf der unmittelbar eine Beschichtung mit eine Affinität zu den ersten Molekülen aufweisenden zweiten Molekulen vorgesehen ist, und wobei die Elektrode mit einem Mittel zur Erzeugung eines elektrischen Felds verbunden ist, so daß in der Lösung eine in bezug zur Elektrode gerichtete Bewegung der ersten Moleküle bewirkbar ist.

Damit wird eine Vorrichtung zur Durchführung des neuerungsgemäßen Verfahrens zur Verfügung gestellt, bei der das nachteilige Zusetzen der Beschichtung vermieden und ein Anreichern spezifischer Moleküle an einer Oberfläche bewirkbar ist. Die Elektrode ist einfach und billig herstellbar; sie kann daher als Einmalartikel verwendet werden.

Die Elektrode ist vorzugsweise aus einem elektrisch leitfähigen Kunststoff, insbesondere einem Polycarbonat, einem Polycarben, Polycarbonat, Polycarbonat-Mischpolymerisat oder Homopolymerisat mit einem leitfähigen Zusatz, wie Graphit, hergestellt. Derartige elektrisch leitfähige Kunststoffe sind bsp. aus der DE 35 41 721 A1 bekannt, deren Inhalt hiermit einbezogen wird.

Das Mittel zur Erzeugung des elektrischen Felds kann ein Mittel zur Erzeugung eines elektrischen Wechselfelds sein. So kann eine schnelle und spezifische Belegung der Beschichtung mit den anzureichernden bzw. aufzureinigenden ersten Molekülen erzielt werden.

Je nachdem, ob das erste Molekül eine positive oder eine negative Ladung trägt, kann das Mittel zur Erzeugung des elektrischen Felds die Elektrode als Anode oder als Kathode umfassen.

Die als Elektrodenmaterial vorliegenden Kunststoffe lassen eine sehr einfache und damit kostengünstige Belegungschemie (Hitoshi Kohsaka : J. Clin. Lab. Anal. 8:452-455 (1994)) für die die Selktionskriterien vorgebenden zweiten Moleküle zu. Die auf der Elektrode vorgesehene Beschichtung weist nach einem weiteren Ausgestaltungsmerkmal mindestens einen aliphatischen Rest auf, der vorzugsweise über eine NH- oder SH-Bindung an die Elektrode gebunden ist. Der aliphatische Rest kann dabei eine Kettenlänge von 2 - 20, vorzugsweise von 6 - 10, Kohlenstoffatomen aufweisen. An das freie Ende des aliphatischen Rests ist zweckmäßigerweise eine Protein-, Peptid- oder eine Nukleotid-Sequenz gebunden. Die Nukleotid-Sequenz kann 5 - 30 Nukleotide umfassen. Dabei ist es vorteilhaft, wenn die Nukleotid-Sequenz ein erstes Oligonukleotid ist, das aus einer Kette von 10 - 20, vorzugsweise von 15 Nukleotiden, gebildet ist. Als besonders vorteilhaft wird angesehen, an das erste Oligonukleotid, vorzugsweise über eine Zuckerphosphat-Bindung, ein zweites Oligonukleotid zu binden.

Die Elektrode kann mit einem Heizelement versehen sein, das durch einen elektrischen Isolator von der Elektrode getrennt ist. Der Isolator kann aus Glas oder Keramik, vorzugsweise aus Aluminiumoxid oder -nitrid, hergestellt sein. Bei dem Heizelement kann es sich um ein aus Platin hergestelltes Widerstandsheizelement handeln. Das Widerstandsheizelement kann in einer Ausgestaltung der Erfindung auch mit der Elektrode identisch sein, da bei geeigneter Schaltung der im Vergleich zu den Zuleitungen hohe elektrische Wiederstand der Kunststoffelektrode zur Erwärmung der Elektrodenoberfläche genutzt werden kann.

Um unerwünschte Moleküle von der Beschichtung abzuführen und eine Neuverteilung von aufzureinigenden und anzureichernden ersten Molekülen in der Lösung zu bewirken, ist zweckmäßigerweise ein Mittel zum Durchmischen der Lösung vorgesehen. Dabei kann es sich um einen elektrisch betreibaren Rührer oder um einen hindurchgeleiteten Gasstrom handeln.

Nach einem weiteren Ausgestaltungsmerkmal der Erfindung sind die elektrischen Größen zur Erzeugung des elektrischen Felds, zum Betrieb des Heizelements sowie des Mittels zum Durchmischen in Abhängigkeit von Parametern der Lösung wie deren ph-Wert, Ionenleitfähigkeit, Konzentration an ersten Molekülen, Temperatur und dgl. vorzugsweise automatisch steuerbar. Zur automatischen Steuerung wird zweckmäßigerweise ein Computer zum Einsatz kommen.

Schließlich wird eine Zusammenstellung von Mitteln zur Herstellung der neuerungsgemäßen Vorrichtung und zur Durchführung des neuerungsgemäßen Verfahrens beansprucht.

### Beispiel 1 - Aufbau der Beschichtung und des darunter befindlichen Körpers

Auf einen Trägerstab ist eine erste Isolationsschicht, die aus Glas oder Keramik, bsp. aus Aluminiumoxid, gebildet sein kann, aufgebracht. Darauf ist eine leitfähige, bsp. in Form eines Platinmäanders hergestellte, Schicht zum Heizen aufgebracht. Die leitfähige Schicht ist wiederum mit einer dünnen zweiten Isolationsschicht abgedeckt. Sie kann eine Dicke von 150 µm aufweisen und aus Glas hergestellt sein. Darauf befindet sich eine aus Gold gebildete Elektrode. Sie ist kontaktiert.

Auf der Elektrode ist die Beschichtung vorgesehen. Über SH-Gruppen sind an die Oberfläche der Elektrode C6 aliphatische Linkermoleküle gebunden. An das freie Ende der Linkermoleküle sind jeweils Spacermoleküle, bsp. aus 10 Thymidinresten bestehende Oligonukleotide, gebunden. An deren freien Enden ist jeweils über eine Zuckerphosphat-Bindung 10 pmol eines 20-mer mit einer vorgegebenen Sequenz gebunden. Die mit der Beschichtung versehene Elektrode wird üblicherweise in Kombination mit der beanspruchten Vorrichtung verwendet. Er kann aber auch ein gesonderter Neuerungsgegenstand sein.

### Beispiel 2 - Aufreinigung

Die im Beispiel 1 beschriebene Elektrode wird in 1 ml einer 20 pmol radioaktiv markiertes Olionukleotid (20-mer) und 40 pmol DNA-Einzelstränge enthaltenden Meßlösung eingetaucht. Das Oligonukleotid ist komplementär zu dem in der Beschichtung gebundenen Oligonukleotid, die DNA-Einzelstränge dagegen nicht. Für die mittels Cherenkov-Zählung ermittelte Aktivität der der Beschichtung werden die folgenden Werte beobachtet:

Wird an die Elektrode eine Spannung von 0,2 V und ein Strom vom 0,8 mA angelegt, so wird nach mehrmaligem Umpolen eine Bindung von 9% der Gesamtaktivität an die Beschichtung beobachtet. Bei zweiminütiger Schaltung der Elektrode als Anode ohne Umpolung beträgt die Bindung der Gesamtativität 2%. - Ohne Anlegen einer Spannung werden weniger als 0,2% der Gesamtaktivität an die Beschichtung gebunden.

Nachfolgend werden Ausführungsbeispiele der Erfindung anhand der Zeichnung erläutert. Hierin zeigen
- Fig. 1: einen schematischen Querschnitt durch ein erstes Ausführungsbeispiel einer Elektrode,
- Fig. 2: einen schematischen Querschnitt durch ein zweites Ausführungsbeispiel einer Elektrode,
- Fig. 3: eine perspektivische Ansicht gemäß Fig. 1,
- Fig. 4: eine perspektivische Ansicht eines Behälters mit Elektrode und Gegenelektrode,
- Fig. 5: eine perspektivische Ansicht eines dritten Ausführungsbeispiels einer Elektrode.

Ein aus Teflon hergestellter Trägerstab 1 ist mit einer leitfähigen Schicht bzw. Elektrode 2, bestehend aus einem elektrisch leitfähigen Kunststoff, versehen. Die der Lösung (hier nicht dargestellt) zugewandte Fläche 3 der Elektrode 2 ist mit Oligonukleotiden 4 beschichtet. Innerhalb des Trägerstabs 1 ist eine erste Leitung 5 eingegossen, die mit der Elektrode 2 kontaktiert ist. Zwei weitere zweite Leitungen 6 (hier mit unterbrochener Linie dargestellt) können zum Beheizen ebenfalls mit der Elektrode 2 kontaktiert sein.

Bei dem in Fig. 2 gezeigten Ausführungsbeispiel ist zusätzlich zwischen der Elektrode 2 und dem Trägerstab 1 eine elektrische isolierende Zwischenschicht 7 vorgesehen. Sie trennt die Elektrode 2 von einer Heizschicht 8, die unmittelbar auf dem aus Teflon hergestellten Trägerstab 1 vorgesehen ist.

Fig. 3 zeigt eine perspektivische Ansicht des in Fig. 1 beschriebenen Ausführungsbeispiels. Mit 9 sind in einer Lösung befindliche ladungstragende erste Moleküle bezeichnet.

Fig. 4 zeigt das Ausführungsbeispiel gemäß Fig. 3 in perspektivischer Ansicht. Der Trägerstab 1 taucht in eine in einem Behälter 10 aufgenommenen Lösung ein, in der sich erste Moleküle 9 befinden. Die erste Leitung 5 ist mit einer Wechselspannungsquelle 11 verbunden. Ebenfalls mit der Wechselspannungsquelle 11 verbunden ist über eine dritte Leitung 12 eine in die Losung eintauchende Gegenelektrode 13

Fig. 5 zeigt in perspektivischer Ansicht ein drittes Ausführungsbeispiel einer Elektrode. An der Spitze des aus isolierendem Kunststoff hergestellten Trägerstabs 1 befindet sich die Fläche 3 der Elektrode 2. Die eingegossene erste Leitung 5 ist mit einer ersten Anschlußbuchse 14 und die zweiten Leitungen 6 jeweils mit zweiten Anschlußbuchsen 15 verbunden. In die Anschlußbuchsen 14 bzw. 15 können Stecker von Anschlußkabeln eingesteckt werden.

Die Funktion der Vorrichtung ist folgende:

Um Biomolekül-Proben aus einer Lösung zu gewinnen, wird eine erfindungsgemäße Vorrichtung in die Lösung getaucht. Sodann wird eine Spannung über die auf dem Trägerstab 1 vorgesehene Elektrode 2 und einer in die Lösung eintauchenden Gegenelektrode 13 angelegt. Das bewirkt je nach Polarisation der Elektrode 2 eine elektrophoretische Wanderung von entgegengesetzt geladenen Biomolekülen in Richtung der Elektrode 2. Bei Erreichen der aus Oligonulkleotiden 4 bestehenden Beschichtung werden diese daran gebunden.

Im Fall von doppelsträngiger DNA wird die Polarisation der Elektrode 2 nach einem vorgegebenen Zeitabschnitt unterbrochen. Es findet dann ein Heizen der Elektrode 2 statt. Dadurch werden die an den Oligonukleotiden 4 anhaftenden DNA Doppelstränge in ihre Komponenten zerlegt. Die Einzelstränge werden durch Wiederanlegen der Polarisation an die Oligonukleotide 4 zurückbewegt und dort gebunden. Auf diese Weise können schnell und einfach Proben aus der Lösung gewonnen werden.

### Bezugszeichenliste

- 1: Trägerstab
- 2: Elektrode
- 3: Fläche
- 4: Oligonukleotide
- 5: erste Leitung
- 6: zweite Leitung
- 7: Zwischenschicht
- 8: Heizschicht
- 9: erste Moleküle
- 10: Behälter
- 11: Wechselspannungsquelle
- 12: dritte Leitung
- 13: Gegenelektrode
- 14: erste Anschlußbuchse
- 15: zweite Anschlußbuchse

## Patentansprüche

1. Verfahren zur Aufreinigung und Anreicherung von ladungstragenden ersten Molekülen (9), wie Proteinen, Nukleinsäuren und dgl., umfassend die folgenden Schritte:
a) Bereitstellen einer die ersten Moleküle (9) enthaltenden Lösung,
b) Inkontaktbringen der Lösung mit einer Elektrode (2), auf der unmittelbar eine Beschichtung mit eine Affinität zu den ersten Molekülen (9) aufweisenden zweiten Molekülen (4) vorgesehen ist und
c) und Verbinden der Elektrode (2) mit einem Mittel (11) zur Erzeugung eines elektrischen Felds, so daß in der Lösung eine in bezug zur Elektode (2) gerichtete Bewegung der ersten Moleküle (9) bewirkt wird.

2. Verfahren nach Anspruch 1, wobei zur Erzeugung des elektrischen Felds eine erste Spannung an die Elektrode (2) angelegt wird, so daß sie als Anode wirkt, an die eine negative Ladung tragende erste Moleküle (9), z.B. Nukleinsäuren, so angezogen werden, daß eine Bindung an die zweiten Moleküle (4) erreicht wird.

3. Verfahren nach Anspruch 1, wobei zur Erzeugung des elektrischen Felds eine erste Spannung an die Elektrode (2) angelegt wird, so daß sie als Kathode wirkt, an die eine positive Ladung tragende erste Moleküle (9), z.B. Proteine, so angezogen werden, daß eine Bindung an die zweiten Moleküle (4) erreicht wird.

4. Verfahren nach einem der Ansprüche 1 oder 2, wobei insbesondere nach dem Binden der ersten an die zweiten Moleküle (4) der folgende Schritt durchgeführt wird:
d₁) Umpolen und Anlegen einer zweiten Spannung, so daß die Elektrode (2) als Kathode wirkt, von der eine negative Ladung tragende erste Moleküle (9), z.B. Nukleinsäuren, abgestoßen werden.

5. Verfahren nach einem der Ansprüche 1 oder 3, wobei insbesondere nach dem Binden der ersten (9) an die zweiten Moleküle (4) der folgende Schritt durchgeführt wird:
d₂) Umpolen und Anlegen einer zweiten Spannung, so daß die Elektrode (2) als Anode wirkt, von der eine positive Ladung tragende erste Moleküle (9), z.B. Proteine, abgestoßen werden.

6. Verfahren nach einem der vorhergehenden Ansprüche, wobei insbesondere während und/oder nach dem Schritt lit. c der folgende Schritt durchgeführt wird:
e) Aufheizen der Elektrode (1) oder der Lösung, so daß die ersten Moleküle (3) thermisch in ihre Komponenten oder Untereinheiten, z.B. einzelsträngige Nukleinsäuren, dissoziiert oder denaturiert werden.

7. Verfahren nach einem der vorhergehenden Ansprüche, wobei insbesondere nach dem Schritt lit. d₁ bzw. d₂ der folgende Schritt durchgeführt wird:
f) Abkühlen der Elektrode (2) oder der Lösung, so daß eine Bindung der ersten Moleküle (9), Komponenten, Untereinheiten und oder an die zweiten Moleküle (4) bewirkt wird.

8. Verfahren nach einem der vorhergehenden Ansprüche, wobei einer oder mehrere der Schritte lit. c - lit. f wiederholt wird/werden.

9. Verfahren nach einem der vorhergehenden Ansprüche, wobei die Lösung vorzugsweise mechanisch durchmischt wird.

10. Verfahren nach einem der Ansprüche 2 - 9, wobei die Maximalwerte der ersten und zweiten Spannung so gewählt werden, daß eine durch Elektrolyse erfolgende Degradierung oder Dissoziation der ersten (9) und zweiten Moleküle (4) sowie der Komponenten und Untereinheiten vermieden wird.

11. Verfahren nach einem der vorhergehenden Ansprüche 4 - 10, wobei der Maximalwert der zweiten Spannung so gewählt wird, daß eine Trennung von zwischen den ersten Molekülen (9) bzw. deren Komponenten oder Untereinheiten und den zweiten Molekülen (4) gebildeten Bindungen vermieden wird.

12. Verfahren nach einem der vorhergehenden Ansprüche, wobei die Maximalwerte der ersten und/oder zweiten Spannung/en, die Dauer der Polung bzw. Umpolung und/oder die Temperatur der Elektrode (2) in Abhängigkeit von Parametern der Losung, wie deren ph-Wert, Ionenleitfähigkeit, Konzentration an ersten Molekülen (9), Temperatur und dgl., vorzugsweise automatisch, gesteuert wird.

13. Vorrichtung zur Anreicherung von ladungstragenden in einer Lösung befindlichen ersten Molekülen (9), wie Nukleinsäuren, Proteinen und dgl., mit einer Elektrode (2) auf der unmittelbar eine Beschichtung mit eine Affinität zu den ersten Molekülen (3) aufweisenden zweiten Molekülen (4) vorgesehen ist, und wobei die Elektrode (2) mit einem Mittel zur Erzeugung eines elektrischen Felds (11) verbunden ist, so daß in der Lösung eine in bezug zur Elektode (2) gerichtete Bewegung der ersten Moleküle (9) bewirkbar ist.

14. Vorrichtung nach Anspruch 13, wobei ein Behälter (10) zur Aufnahme einer die ersten Moleküle (9) enthaltenden Lösung vorgesehen ist.

15. Vorrichtung nach Anspruch 13 oder 14, wobei das Mittel (11) zur Erzeugung des elektrischen Felds ein Mittel zur Erzeugung eines elektrischen Wechselfelds ist.

16. Vorrichtung nach einem der Ansprüche 13 bis 15, wobei das Mittel (11) zur Erzeugung des elektrischen Felds die Elektrode (2) als Anode umfaßt.

17. Vorrichtung nach einem Ansprüche 13 - 16, wobei das Mittel (11) zur Erzeugung des elektrischen Felds die Elektrode (2) als Kathode umfaßt.

18. Vorrichtung nach einem der Ansprüche 13 - 17, wobei die Elektrode (2) eine aus Gold, einer Gold/Platin- oder einer Platinlegierung hergestellt ist.

19. Vorrichtung nach einem der Ansprüche 13 - 17, wobei die Elektrode (2) aus aus einem leitfähigen Kunststoff, wie einem Polycarbonat, Polycarbonat-Mischpolymerisat oder einem Homopolymerisat mit einem leitfähigen Zusatz, wie Graphit, hergestellt ist.

20. Vorrichtung nach einem der Ansprüche 13 - 19, wobei die Beschichtung mindestens einen aliphatischen Rest aufweist, der vorzugsweise über eine NH- oder SH-Bindung an die Elektrode (2) gebunden ist.

21. Vorrichtung nach Anspruch 20, wobei der aliphatische Rest eine Kettenlänge von 2 - 20, vorzugsweise von 6 - 10, Kohlenstoffatomen aufweist.

22. Vorrichtung nach Anspruch 20 oder 21, wobei an das freie Ende des aliphatischen Rests eine Protein- oder Peptidsequenz gebunden ist.

23. Vorrichtung nach einem der Ansprüche 13 bis 22, wobei an das freie Ende des aliphatischen Rests eine Nukleotid-Sequenz gebunden ist.

24. Vorrichtung nach Anspruch 23, wobei die Nukleotid-Sequenz 5 - 30 Nukleotide umfaßt.

25. Vorrichtung nach einem der Ansprüche 13 bis 24, wobei die Nukleotid-Sequenz ein erstes Oligonukleotid ist, das aus einer Kette von 10 - 20, vorzugsweise von 15 Nukleotiden, gebildet ist.

26. Vorrichtung nach Anspruch 25, wobei das erste Oligonukleotid, vorzugsweise über eine Zuckerphosphat-Bindung, an ein zweites Oligonukleotid gebunden ist.

27. Vorrichtung nach einem der Ansprüche 13 bis 26, wobei die Elektrode (2) auf einem Träger (1) vorgesehen ist.

28. Vorrichtung nach einem der Ansprüche 13 bis 27, wobei der der Träger (1) aus einem elektrisch isolierenden Material, vorzugsweise einem elektrisch isolierenden Kunststoff, hergestellt ist.

29. Vorrichtung nach einem der vorhergehenden Ansprüche 13 - 28, wobei die Elektrode (2) mit einem Heizelement (8) versehen ist, das durch einen elektrischen Isolator (6) von der Elektrode (2) getrennt ist.

30. Vorrichtung nach Anspruch 29, wobei der elektrische Isolator (6) aus Glas oder Keramik, vorzugsweise aus Aluminiumoxid oder -nitrid, hergestellt ist.

31. Vorrichtung nach einem der vorhergehenden Ansprüche 13 - 30, wobei ein Mittel zum Durchmischen der Lösung vorgesehen ist.

32. Vorrichtung nach Anspruch 31, wobei das Mittel zum Durchmischen ein elektrisch betreibarer Rührer ist.

33. Vorrichtung nach einem der Ansprüche 13 - 32, wobei die elektrischen Größen zur Erzeugung des elektrischen Felds, zum Betrieb des Heizelements sowie des Mittels zum Durchmischen in Abhängigkeit von Parametern der Lösung wie deren ph-Wert, Ionenleitfähigkeit, Konzentration an ersten Molekülen (9), Temperatur und dgl., vorzugsweise automatisch, steuerbar sind.

34. Zusammenstellung von Mitteln zur Herstellung der Vorrichtung nach einem der Anspüche 13 bis 33.

35. Zusammenstellung von Mitteln zur Durchführung des Verfahrens nach einem der Anspüche 1 bis 12.

## Claims

1. Process for purifying and concentrating charge-bearing first molecules (9), such as proteins, nucleic acids and the like, comprising the following steps:
a) preparation of a solution containing the first molecules (9),
b) contacting the solution with an electrode (2) which is directly provided with a coating of second molecules (4) having affinity for the first molecules (9), and
c) connecting the electrode (2) to a means (11) for generating an electric field to bring about a movement of the first molecules (9) in the solution directed relative to the electrode (2).

2. Process according to Claim 1, wherein to generate the electric field a first voltage is applied to the electrode (2) so that it acts as anode to which the first molecules (9) bearing a negative charge, for example nucleic acids, are attracted so that binding to the second molecules (4) is achieved.

3. Process according to Claim 1, wherein to generate the electric field a first voltage is applied to the electrode (2) so that it acts as cathode to which the first molecules (9) bearing a positive charge, for example proteins, are attracted so that binding to the second molecules (4) is achieved.

4. Process according to either of Claims 1 or 2, wherein the following step is carried out in particular after the binding of the first to the second molecules (4):
d₁) reversing the polarity and applying a second voltage so that the electrode (2) acts as cathode from which first molecules (9) bearing a negative charge, for example nucleic acids, are repelled.

5. Process according to either of Claims 1 or 3, wherein the following step is carried out in particular after the binding of the first (9) to the second molecules (4):
d₂)reversing the polarity and applying a second voltage so that the electrode (2) acts as anode from which first molecules (9) bearing a positive charge, for example proteins, are repelled.

6. Process according to any of the preceding claims, wherein the following step is carried out in particular during and/or after step c:
e) heating the electrode (2) or the solution so that the first molecules (9) are thermally dissociated or denatured into their components or subunits, for example single-stranded nucleic acids.

7. Process according to any of the preceding claims, wherein the following step is carried out in particular after step d₁ or d₂:
f) cooling the electrode (2) or the solution to bring about binding of the first molecules (9), components and/or subunits to the second molecules (4).

8. Process according to any of the preceding claims, wherein one or more of steps c-f is/are repeated.

9. Process according to any of the preceding claims, wherein the solution is preferably mixed mechanically.

10. Process according to any of Claims 2-9, wherein the maximum values of the first and second voltage are chosen so that degradation or dissociation of the first (9) and second molecules (4), and the. components and subunits, by electrolysis is avoided.

11. Process according to any of the preceding Claims 4-10, wherein the maximum value of the second voltage is chosen so that breaking of linkages formed between the first molecules (9) or their components or subunits and the second molecules (4) is avoided.

12. Process according to any of the preceding claims, wherein the maximum values of the first and/or second voltage(s), the duration of the polarity and reverse polarity and/or the temperature of. the electrode (2) are controlled, preferably automatically, as a function of parameters of the solution such as its pH, ionic conductivity, concentration of first molecules (9), temperature and the like.

13. Apparatus for concentrating charge-bearing first molecules (9) present in a solution, such as nucleic acids, proteins and the like, is provided with an electrode (2) which is directly provided with a coating of second molecules (4) having affinity for the first molecules (9), and where the electrode (2) is connected to a means for generating an electric field (11) so that it is possible to bring about a movement of the first molecules (9) in the solution directed relative to the electrode (2).

14. Apparatus according to Claim 13, wherein a container (10) is provided to accommodate a solution containing the first molecules (9).

15. Apparatus according to Claim 13 or 14, wherein the means (11) for generating the electric field is a means for generating an alternating electric field.

16. Apparatus according to any of Claims 13 to 15, wherein the means (11) for generating the electric field comprises electrode (2) as anode.

17. Apparatus according to any of Claims 13-16, wherein the means (11) for generating the electric field comprises electrode (2) as cathode.

18. Apparatus according to any of Claims 13-17, wherein electrode (2) is produced from gold, a gold/platinum alloy or a platinum alloy.

19. Apparatus according to any of Claims 13-17, wherein electrode (2) is produced from a conducting plastic such as a polycarbonate, polycarbonate copolymer or a homopolymer with a conducting additive such as graphite.

20. Apparatus according to any of Claims 13-19, wherein the coating has at least one aliphatic radical which is preferably linked via an NH or SH linkage to electrode (2).

21. Apparatus according to Claim 20, wherein the aliphatic radical has a chain length of 2-20, preferably of 6-10, carbon atoms.

22. Apparatus according to Claim 20 or 21, wherein a protein or peptide sequence is linked to the free end of the aliphatic radical.

23. Apparatus according to any of Claims 13 to 22, wherein a nucleotide sequence is linked to the free end of the aliphatic radical.

24. Apparatus according to Claim 23, wherein the nucleotide sequence comprises 5-30 nucleotides.

25. Apparatus according to any of Claims 13 to 24, wherein the nucleotide sequence is a first oligonucleotide formed from a chain of 10-20, preferably 15, nucleotides.

26. Apparatus according to Claim 25, wherein the first oligonucleotide is linked, preferably via a sugar-phosphate linkage, to a second oligonucleotide.

27. Apparatus according to any of Claims 13 to 26, wherein the electrode (2) is provided on a support (1).

28. Apparatus according to any of Claims 13 to 27, wherein the support (1) is produced from an electrically insulating material, preferably an electrically insulating plastic.

29. Apparatus according to any of preceding Claims 13-28, wherein electrode (2) is provided with a heating element (8) which is separated from the electrode (2) by an electrical insulator (6).

30. Apparatus according to Claim 29, wherein the electrical insulator (6) is produced from glass or ceramic, preferably from aluminium oxide or nitride.

31. Apparatus according to any of preceding Claims 13-30, wherein a means for mixing the solution is provided.

32. Apparatus according to Claim 31, wherein the means for mixing is a stirrer which can be operated electrically.

33. Apparatus according to any of Claims 13-32, wherein the electrical quantities for generating the electric field and for operating the heating element and the means for mixing can be controlled, preferably automatically, as a function of parameters of the. solution such as its pH, ionic conductivity, concentration of first molecules (9), temperature and the like.

34. Combination of means for producing the apparatus according to any of Claims 13 to 33.

35. Combination of means for carrying out the process according to any of Claims 1 to 12.

## Revendications

1. Procédé de purification et d'enrichissement de premières molécules portant une charge (9), comme des protéines, des acides nucléiques et assimilés, comprenant les étapes suivantes :
a) préparation d'une solution contenant les premières molécules (9);
b) mise en contact de la solution avec une électrode (2), sur laquelle un revêtement des deuxièmes molécules (4) présentant une affinité pour les premières molécules (9) est directement prévu, et
c) connexion de l'électrode (2) avec un moyen (11) pour la production d'un champ électrique, de sorte qu'un mouvement des premières molécules (9) dirigé par rapport à l'électrode (2) est amené dans la solution.

2. Procédé suivant la revendication 1, dans lequel, pour produire le champ électrique, on établit une première tension sur l'électrode (2) de sorte qu'elle agisse en tant qu'anode, sur laquelle des premières molécules portant une charge négative (9), par exemple des acides nucléiques, sont attirées de telle sorte qu'on réalise une liaison avec les deuxièmes molécules (4).

3. Procédé suivant la revendication 1, dans lequel, pour produire le champ électrique, on établit une première tension sur l'électrode (2) de sorte qu'elle agisse en tant que cathode, sur laquelle des premières. molécules portant une charge positive (9), par exemple des protéines, sont attirées de telle sorte qu'on réalise une liaison avec les deuxièmes molécules (4).

4. Procédé suivant l'une des revendications 1 ou 2, dans lequel en particulier après la liaison des premières avec les deuxièmes molécules (4), on réalise l'étape suivante :
d₁) inversion des pôles et établissement d'une deuxième tension, de sorte que l'électrode (2) agisse en tant que cathode, de laquelle des premières molécules portant une charge négative (9), par exemple des acides nucléiques, sont repoussées.

5. Procédé suivant l'une des revendications 1 ou 3, dans lequel en particulier après la liaison des premières (9) avec les deuxièmes molécules (4), on réalise l'étape suivante :
d₂) inversion des pôles et établissement d'une deuxième tension, de sorte que l'électrode (2) agisse en tant qu'anode, de laquelle des premières molécules portant une charge positive (9), par exemple des protéines, sont repoussées.

6. Procédé suivant l'une des revendications précédentes, dans lequel, en particulier pendant et/ou après l'étape c, on réalise l'étape suivante :
e) chauffage de l'électrode (2) ou de la solution, de sorte que les premières molécules (9) soient thermiquement dissociées ou dénaturées en leurs composants ou sous-unités, par exemple des acides nucléiques monocaténaires.

7. Procédé suivant l'une des revendications précédentes, dans lequel, en particulier après l'étape d₁ ou, selon le cas, d₂, on réalise, l'étape suivante :
f) refroidissement de l'électrode (2) ou de la solution, de sorte qu'il se produise une liaison des premières molécules (9), composants et/ou sous-unités avec les deuxièmes molécules (4).

8. Procédé suivant l'une des revendications précédentes, dans lequel on répète l'une ou plusieurs des étapes c à f.

9. Procédé suivant l'une des revendications précédentes, dans lequel la solution est intimement mélangée, de préférence mécaniquement.

10. Procédé suivant l'une des revendications 2 à 9, dans lequel les valeurs maximales des première et deuxième tensions sont choisies de sorte qu'on évite une dégradation ou une dissociation suite à l'électrolyse des premières (9) et deuxièmes molécules (4) ainsi que des composants et sous-unités.

11. Procédé suivant l'une des revendications précédentes 4 à 10, dans lequel la valeur maximale de la deuxième tension est choisie de telle sorte qu'on évite une séparation des liaisons formées entre les premières molécules (9) ou, selon le cas, leurs composants ou sous-unités, et les deuxièmes molécules (4).

12. Procédé suivant l'une des revendications précédentes, dans lequel les valeurs maximales de la ou des première, et/ou deuxième tensions, la durée de la polarité ou, selon le cas, de l'inversion de pôle et/ou la température de l'électrode (2) sont contrôlées, de préférence automatiquement, en fonction de paramètres de la solution, tels que son pH, sa conductivité ionique, sa concentration en premières molécules (9), sa température et assimilés.

13. Dispositif d'enrichissement de premières molécules (9) portant une charge se trouvant dans une solution, telles que des acides nucléiques, des protéines, et assimilés, avec une électrode (2) sur laquelle un revêtement comportant des deuxièmes molécules (4) présentant une affinité pour les premières molécules (9), est directement prévu et dans lequel l'électrode (2) est connectée à un moyen de production d'un champ électrique (11), de sorte qu'un mouvement des premières molécules (9) dirigé par rapport à l'électrode (2) soit possible dans la solution.

14. Dispositif suivant la revendication 13, dans lequel un récipient (10) est prévu pour recevoir une solution contenant les premières molécules (9).

15. Dispositif suivant la revendication 13 ou 14, dans lequel le moyen (11) de production du champ électrique est un moyen de production d'un champ électrique alternatif.

16. Dispositif suivant l'une des revendications 13 à 15, dans lequel le moyen (11) de production du champ électrique comprend l'électrode (2) en tant qu'anode.

17. Dispositif suivant l'une des revendications 13 à 16, dans lequel le moyen (11) de production du champ électrique comprend l'électrode (2) en tant que cathode.

18. Dispositif suivant l'une des revendications 13 à 17, dans lequel l'électrode (2) est fabriquée à partir d'or, d'un alliage d'or/platine ou d'un alliage de platine.

19. Dispositif suivant l'une des revendications 13 à 17, dans lequel l'électrode (2) est fabriquée à partir d'un plastique conducteur, tel qu'un polycarbonate, un copolymère de polycarbonate ou un homopolymère avec un additif conducteur, tel que le graphite.

20. Dispositif suivant l'une des revendications 13 à 19, dans lequel le revêtement présente au moins un radical aliphatique, qui est lié de préférence par une liaison NH ou SH à l'électrode (2).

21. Dispositif suivant la revendication 20, dans lequel le radical aliphatique présente une longueur de chaîne de 2 à 20, de préférence de 6 à 10 atomes de carbone.

22. Dispositif suivant la revendication 20 ou 21, dans lequel une séquence protéique ou peptidique est liée à l'extrémité libre du radical aliphatique.

23. Dispositif suivant l'une des revendications 13 à 22, dans lequel une séquence de nucléotides est liée à l'extrémité libre du radical aliphatique.

24. Dispositif suivant la revendication 23, dans lequel la séquence de nucléotides comprend 5 à 30 nucléotides.

25. Dispositif suivant l'une des revendications 13 à 24, dans lequel la séquence de nucléotides est un premier oligonucléotide formé d'une chaîne de 10 à 20, de préférence de 15 nucléotides.

26. Dispositif suivant la revendication 25, dans lequel le premier oligonucléotide est lié à un deuxième oligonucléotide, de préférence par une liaison sucre-phosphate.

27. Dispositif suivant l'une des revendications 13 à 26, dans lequel l'électrode (2) est prévue sur un support (1).

28. Dispositif suivant l'une des revendications 13 à 27, dans lequel le support (1) est fabriqué à partir d'un matériau isolant de l'électricité, de préférence d'un plastique isolant de l'électricité.

29. Dispositif suivant l'une des revendications précédentes 13 à 28, dans lequel l'électrode (2) est pourvue d'un élément chauffant (8) qui est séparé de l'électrode (2) par un isolateur électrique (6).

30. Dispositif suivant la revendication 29, dans lequel l'isolateur électrique (6) est fabriqué à partir de verre ou de céramique, de préférence d'oxyde ou de nitrure d'aluminium.

31. Dispositif suivant l'une des revendications précédentes de 13 à 30, dans lequel un moyen de mélange intime de la solution est prévu.

32. Dispositif suivant la revendication 31, dans lequel le moyen de mélange intime est un agitateur à fonctionnement électrique.

33. Dispositif suivant l'une des revendications 13 à 32, dans lequel les grandeurs électriques pour la production du champ électrique, pour le fonctionnement de l'élément chauffant et du moyen de mélange intime sont contrôlables, de préférence automatiquement, en fonction de paramètres de la solution tels que son pH, sa conductivité ionique, sa concentration en premières molécules (9), sa température, et assimilés.

34. Combinaison de moyens pour fabriquer le dispositif suivant l'une des revendications 13 à 33.

35. Combinaison de moyens pour réaliser le procédé suivant l'une des revendications 1 à 12.
